(19) Europäisches Patentamt
European Patent Office
Office européen des brevets

(11) Veröffentlichungsnummer : **0 017 066**
**B1**

(12) # EUROPÄISCHE PATENTSCHRIFT

(45) Veröffentlichungstag der Patentschrift : **28.04.82**

(51) Int. Cl.³ : **C 07 D231/20**, C 07 D231/22, C 07 D231/24, A 01 N 47/18

(21) Anmeldenummer : **80101393.9**

(22) Anmeldetag : **17.03.80**

(54) **N,N-Dimethyl-O-pyrazolyl-carbaminsäureester, Verfahren zu ihrer Herstellung und ihre Verwendung als Schädlingsbekämpfungsmittel.**

(30) Priorität : **29.03.79 DE 2912494**

(43) Veröffentlichungstag der Anmeldung : **15.10.80 (Patentblatt 80/21)**

(45) Bekanntmachung des Hinweises auf die Patenterteilung : **28.04.82 Patentblatt 82/17**

(84) Benannte Vertragsstaaten : **AT BE CH DE FR GB IT NL SE**

(56) Entgegenhaltungen :
**EP - A - 0 005 240**
**EP - A - 0 009 634**
**DE - A - 2 644 588**
**DE - A - 2 644 589**
**DE - A - 2 721 188**

(73) Patentinhaber : **BAYER AG**
**Zentralbereich Patente, Marken und Lizenzen**
**D-5090 Leverkusen 1, Bayerwerk (DE)**

(72) Erfinder : **Maurer, Fritz, Dr.**
**Roeberstrasse 8**
**D-5600 Wuppertal 1 (DE)**
Erfinder : **Schröder, Rolf, Dr.**
**Pahlkestrasse 17**
**D-5600 Wuppertal 1 (DE)**
Erfinder : **Homeyer, Bernhard, Dr.**
**Obere Strasse 28**
**D-5090 Leverkusen 3 (DE)**
Erfinder : **Hammann, Ingeborg, Dr.**
**Belfortstrasse 9**
**D-5000 Köln (DE)**

**EP 0 017 066 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

N,N-Dimethyl-O-pyrazolyl-carbaminsäureester, Verfahren zu ihrer Herstellung und ihre Verwendung als Schädlingsbekämpfungsmittel

Die Erfindung betrifft neue N,N-Dimethyl-O-pyrazolylcarbaminsäureester, mehrere Verfahren zu ihrer Herstellung sowie ihre Verwendung als Schädlingsbekämpfungsmittel, insbesondere als Insektizide.

Es ist bekannt, daß bestimmte N,N-Dimethyl-O-pyrazolylcarbaminsäureester, wie z.B. N,N-Dimethyl-O-)1-phenyl-3-methyl-pyrazol(5)yl)- und N,N-Dimethyl-O-(1(isopropyl-3-methyl-pyrazol(5)yl)-carbaminsäureester, insektizide Eingenschaften aufweisen (vergleiche CH-PS 279 553). Die insektizide Wirkung dieser bekannten Verbindungen ist jedoch, insbesondere bei niedrigen Wirkstoffkonzentrationen und Aufwandmengen, nicht immer zufriedenstellend.

Aus DE-A 2 721 188, 2 644 588, 2 644 589 sind ebenfalls N,N-Dimethyl-O-pyrazolyl-carbaminsäureester bekannt, deren Wirkung jedoch bei niedrigen Wirkstoffkonzentrationen nicht immer befriedigt.

Es wurden nun neue N,N-Dimethyl-O-pyrazolyl-carbaminsäureester der Formel I

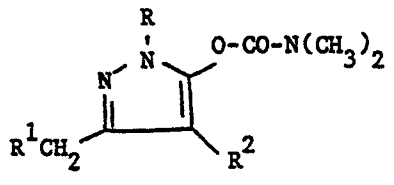

(I)

gefunden, in welcher

R für Alkyl, Cyanoäthyl, Cycloalkyl oder Phenyl steht,

$R^1$ für Alkoxy, Alkylthio, Alkylsulfinyl oder Alkylsulfonyl steht und

$R^2$ für Alkyl steht.

Man erhält die N,N-Dimethyl-O-pyrazolyl-carbaminsäureester der Formel (I), wenn man

a) 5-Hydroxy-pyrazole der Formel II

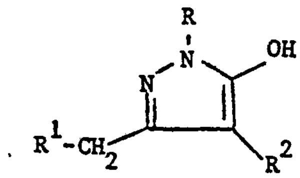

(II)

in welcher

R, $R^1$ und $R^2$ die oben angegebene Bedeutung haben, mit N,N-Dimethyl-carbaminsäurehalogeniden der Formel III

$$Hal—CO—N(CH_3)_2$$

in welcher

Hal für Chlor oder Brom steht,

gegebenenfalls in Gegenwart eines Säureakzeptors und gegebenenfalls unter Verwendung eines inerten Verdünnungsmittels umsetzt, oder

b) 5-Hydroxy-pyrazole der Formel (II), in welcher R, $R^1$ und $R^2$ die oben angegebene Bedeutung haben,

mit Phosgen und anschließend mit Dimethylamin, gegebenenfalls in Gegenwart eines Säureakzeptors und gegebenenfalls unter Verwendung eines inerten Verdünnungsmittels umsetzt.

Verbindungen der Formel (I), in welcher R und $R^2$ die oben angegebene Bedeutung haben und $R^1$ für Alkylsulfinyl steht, erhält man auch, indem man

c) N,N-Dimethyl-O-(3-alkylthiomethyl-pyrazol(5)yl)-carbaminsäureester der Formel (I), in welcher R und $R^2$ die oben angegebene Bedeutung haben und $R^1$ für Alkylthio steht,

mit mindestens der äquimolaren Menge Wasserstoffperoxid, gegebenenfalls unter Verwendung eines Verdünnungsmittels umsetzt.

Verbindungen der Formel (I), in welcher R und $R^2$ die oben angegebene Bedeutung haben und $R^1$ für Alkylsulfonyl steht, erhält man auch, indem man

d) N,N-Dimethyl-O-(3-alkylthiomethyl-pyrazol(5)yl)-carbaminsäureester der Formel (I), in welcher R und

$R^2$ die oben angegebene Bedeutung haben und $R^1$ für Alkylthio steht,

mit wenigstens zwei Moläquivalenten m-Chlor-perbenzoesäure, gegebenenfalls in Gegenwart eines Verdünnungsmittels umsetzt.

Die neuen N,N-Dimethyl-O-pyrazolyl-carbaminsäureester der Formel (I) zeichnen sich durch hohe Wirksamkeit als Schädlingsbekämpfungsmittel, insbesondere durch ihre hervorragende insektizide Wirkung aus.

Überraschenderweise zeigen die erfindungsgemäßen N,N-Dimethyl-O-pyrazolyl-carbaminsäureester eine erheblich höhere insektizide Wirkung als die aus dem Stand der Technik vorbekannten Verbindungen analoger Konstitution und gleicher Wirkungsrichtung.

Die Erfindung betrifft vorzugsweise Verbindungen der Formel (I), in welcher

R für geradkettiges oder verzweigtes Alkyl mit 1 bis 8, insbesondere mit 1 bis 5 Kohlenstoffatomen, für Cyanoäthyl oder für Cycloalkyl mit 3 bis 6 Kohlenstoffatomen steht,

$R^1$ für Alkoxy, Alkylthio, Alkylsulfinyl oder Alkylsulfonyl, jeweils mit geradkettigem oder verzweigtem Alkylrest mit 1 bis 5, insbesondere mit 1 bis 3 Kohlenstoffatomen steht und

$R^2$ für geradkettiges oder verzweigtes Alkyl mit 1 bis 5, insbesondere mit 1 bis 3 Kohlenstoffatomen steht.

Verwendet man beispielsweise bei Verfahren (a) 1-Methyl-3-äthylsulfinylmethyl-4-iso-propyl-5-hydroxy-pyrazol und Dimethylcarbaminsäurechlorid, bei Verfahren (b) 1-Cyanoäthyl-3-methylsulfonylmethyl-4-äthyl-5-hydroxypyrazol, Phosgen und Dimethylamin, bei Verfahren (c) O-(1-n-Propyl-3-methylthiomethyl-4n-propyl-pyrazol(5)yl)-N,N-dimethyl-carbaminsäureester und Wasserstoffperoxid, und bei Verfahren (d) O-(1-Cyclopropyl-3-methylthiomethyl-4-äthyl-pyrazol(5)yl)-N,N-dimethyl-carbaminsäureester und m-Chlor-perbensoesäure als Ausgangsstoffe, so können die entsprechenden Reaktionen durch folgende Formelschemata skizziert werden :

(a)

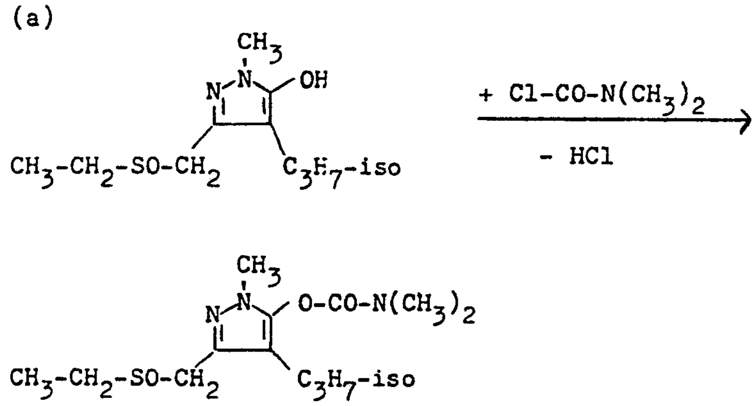


(b)

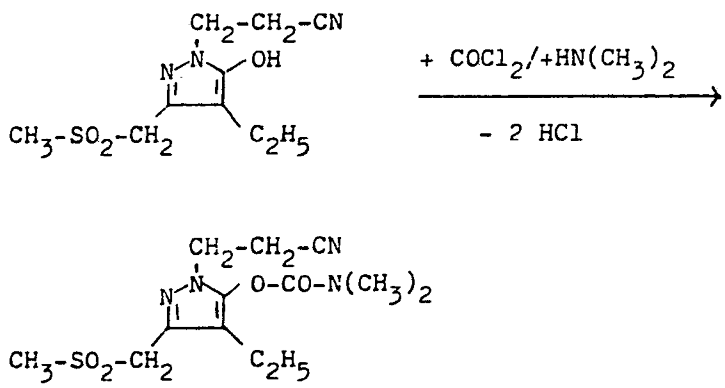


(c)

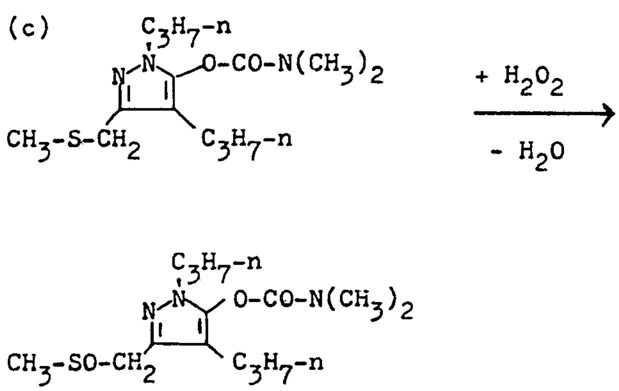

(d)

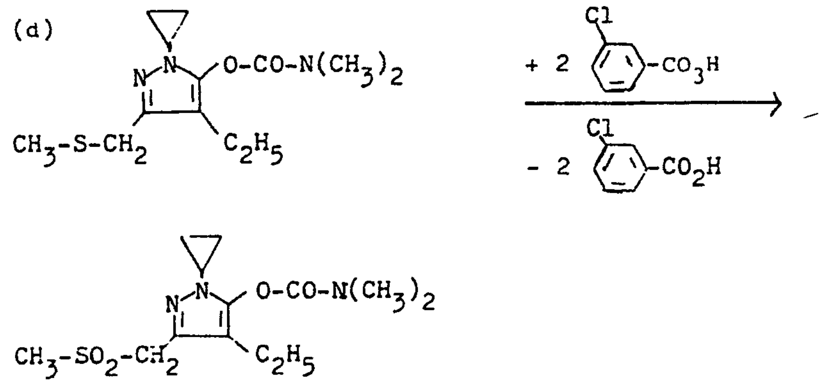

Die bei den Verfahren (a) und (b) als Ausgangsstoffe zu verwendenden 5-Hydroxy-pyrazole sind durch formel (II) definiert. Vorzugsweise stehen darin R,$R^1$ und $R^2$ für diejenigen Reste, welche bei der Definition der Reste R,$R^1$ und $R^2$ in Formel (I) als bevorzugt genannt wurden.

5-Hydroxy-pyrazole der Formel (II) können nach im Prinzip bekannten Verfahren hergestellt werden (vergleiche DE-OS 2 644 588). Man erhält sie beispielsweise durch Umsetzung von γ-Alkoxy-2-alkyl- bzw. γ-Alkylthio-2-alkyl-acetessigsäurealkylestern mit Hydrazinderivaten $H_2N$-NHR (R hat die oben angegebene Bedeutung) bei Temperaturen zwischen O und 100 °C, vorzugsweise zwischen 20 und 80 °C, gegebenenfalls unter Verwendung eines Verdünnungsmittels wie z.B. Methanol.

Die 3-Alkylthiomethyl-5-hydroxy-pyrazole können nach bekannten Methoden mit Wasserstoffperoxid zu den entsprechenden Alkylsulfinylverbindungen bzw. mit m-Chlorperbenzoesäure zu den entsprechenden Alkylsulfonylverbindungen oxidiert werden (siehe Herstellungsverfahren (c) und (d)).

Die als Vorprodukte für die Herstellung der neuen Verbindungen der Formel (I) einzusetzenden γ-Alkoxy-2-alkyl- bzw. γ-Alkylthio-2-alkyl-acetessigsäurealkyl-ester erhält man durch Umsetzung von γ-Alkoxy- bzw. von γ-Alkylthioacetessigsäureestern mit Alkylierungsmitteln wie Brom- oder Jod-alkanen oder Dialkylsulfaten in Gegenwart einer Base wie z.B. Kalium-tert.-butylat, gegebenenfalls unter Verwendung eines Verdünnungsmittels, wie z.B. Tetrahydrofuran, bei Temperaturen zwischen 0 und 100 °C, vorzugsweise zwischen 20 und 80 °C.

γ-Alkoxy- bzw. γ-Alkylthio-acetessigester, welche hierbei als Ausgangsstoffe eingesetzt werden, sind bekannt (vergleiche DE-OS 2 644 588).

Als Beispiele für die Ausgangsstoffe der Formel (II) seien genannt:

3-Methoxymethyl-, 3-Äthoxymethyl-, 3-n-Propoxymethyl-, 3-iso-Propoxymethyl-, 3-Methylthiomethyl-, 3-Äthylthiomethyl-, 3-n-Propylthiomethyl-, 3-iso-Propylthiomethyl-, 3-Methylsulfinylmethyl-, 3-Äthylsulfinylmethyl-, 3-n-Propylsulfinylmethyl-, 3-iso-Propylsulfinylmethyl-, 3-Methylsulfonylmethyl-, 3-Äthylsulfonylmethyl-, 3-n-Propylsulfonylmethyl- und 3-iso-Propylsulfonylmethyl-4-methyl-, -4-äthyl-, -4-n-propyl- und -4-iso-propyl-1-methyl-5-hydroxy-pyrazol;

3-Methoxy-methyl-, 3-Äthoxymethyl-, 3-n-Propoxymethyl-, 3-iso-Propoxymethyl-, 3-Methylthiomethyl-, 3-Äthylthiomethyl-, 3-n-Propylthiomethyl-, 3-iso-Propylthiomethyl-, 3-Methylsulfinylmethyl-, 3-Äthylsulfinylmethyl-, 3-n-Propylsulfinylmethyl-, 3-iso-Propylsulfinylmethyl-, 3-Methylsulfonylmethyl-, 3-Äthylsulfonylmethyl-, 3-n-Propylsulfonylmethyl- und 3-iso-Propylsulfonylmethyl-, -4-methyl-, -4-äthyl-, -4-n-propyl- und -4-isopropyl-, -1-äthyl-5-hydroxy-pyrazol;

3-Methoxymethyl-, 3-Äthoxymethyl-, 3-n-Propoxymethyl-, 3-iso-Propoxymethyl-, 3-Methylthiomethyl-, 3-Äthylthiomethyl-, 3-n-Propylthiomethyl-, 3-iso-Propylthiomethyl-, 3-Methylsulfinylmethyl-, 3-Äthylsulfinylmethyl-, 3-n-Propylsulfinylmethyl-, 3-iso-Propylsulfinylmethyl-, 3-Methylsulfonylmethyl-, 3-Äthylsulfonylmethyl-, 3-n-Propylsulfonylmethyl- und 3-iso-Propylsulfonylmethyl-, 4-methyl-, -4-äthyl-, -4-n-propyl- und -4-iso-propyl-1-n-propyl-5-hydroxy-pyrazol;

3-Methoxymethyl-, 3-Äthoxymethyl-, 3-n-Propoxymethyl-, 3-iso-Propoxymethyl-, 3-Methylthiomethyl-, 3-Äthylthiomethyl-, 3-n-Propylthiomethyl-, 3-iso-Propylthiomethyl-, 3-Methylsulfinylmethyl-, 3-Äthylsulfinylmethyl-, 3-n-Propylsulfinylmethyl-, 3-iso-Propylsulfinylmethyl-, 3-Methylsulfonylmethyl-, 3-Äthylsulfonylmethyl-, 3-n-Propylsulfonylmethyl- und 3-iso-Propylsulfonylmethyl-4-methyl-, -4-äthyl-, -4-n-propyl- und -4-iso-propyl-1-iso-propyl-5-hydroxy-pyrazol;

3-Methoxymethyl-, 3-Äthoxymethyl-, 3-n-Propoxymethyl-, 3-iso-Propoxymethyl-, 3-Methylthiomethyl-, 3-Äthylthiomethyl-, 3-n-Propylthiomethyl-, 3-iso-Propylthiomethyl-, 3-Methylsulfinylmethyl-, 3-Äthylsulfinylmethyl-, 3-n-Propylsulfinylmethyl-, 3-iso-Propylsulfinylmethyl-, 3-Methylsulfonylmethyl-, 3-Äthylsulfonylmethyl-, 3-n-Propylsulfonylmethyl- und 3-iso-Propylsulfonylmethyl-4-methyl-, -4-äthyl-, -4-n-propyl- und -4-iso-propyl-1-sek.-butyl-5-hydroxy-pyrazol;

3-Methoxymethyl-, 3-Äthoxymethyl-, 3-n-Propoxymethyl-, 3-iso-Propoxymethyl-, 3-Methylthiomethyl-, 3-Äthylthiomethyl-, 3-n-Propylthiomethyl-, 3-iso-Propylthiomethyl-, 3-Methylsulfinylmethyl-, 3-Äthylsulfinylmethyl-, 3-n-Propylsulfinylmethyl-, 3-iso-Propylsulfinylmethyl-, 3-Methylsulfonylmethyl-, 3-Äthylsulfonylmethyl-, 3-n-Propylsulfonylmethyl- und 3-iso-Propylsulfonylmethyl-4-methyl-, -4-äthyl-, -4-n-propyl- und -4-iso-propyl-1-tert.-pentyl-5-hydroxy-pyrazol;

3-Methoxy-methyl, 3-Äthoxymethyl-, 3-n-Propoxymethyl-, 3-iso-Propoxymethyl-, 3-Methylthiomethyl-, 3-Äthylthiomethyl-, 3-n-Propylthiomethyl-, 3-iso-Propylthiomethyl-, 3-Methylsulfinylmethyl-, 3-Äthylsulfinylmethyl-, 3-n-Propylsulfinylmethyl-, 3-iso-Propylsulfinylmethyl-, 3-Methylsulfonylmethyl-, 3-Äthylsulfonylmethyl-, 3-n-Propylsulfonylmethyl- und 3-iso-Propylsulfonylmethyl-4-methyl-, 4-äthyl-, 4-n-propyl- und -4-iso-propyl-1-(2-cyano-äthyl)-5-hydroxy-pyrazol ;

3-Methoxymethyl-, 3-Äthoxymethyl-, 3-n-Propoxymethyl-, 3-iso-Propoxymethyl-, 3-Methylthiomethyl-, 3-Äthylthiomethyl-, 3-n-Propylthiomethyl-, 3-iso-Propylthiomethyl-3-Methylsulfinylmethyl-, 3-Äthylsulfinylmethyl-, 3-n-Propylsulfinylmethyl-, 3-iso-Propylsulfinylmethyl-, 3-Methylsulfonylmethyl-, 3-Äthylsulfonylmethyl-, 3-n-Propylsulfonylmethyl- und 3-iso-Propylsulfonylmethyl-4-methyl-, 4-athyl-, -4-n-propyl- und -4-iso-propyl-1-cyclopropyl-5-hydroxy-pyrazol ;

3-Methoxymethyl-, 3-Äthoxymethyl-, 3-n-Propoxymethyl-, 3-iso-Propoxymethyl-, 3-Methylthiomethyl-, 3-Äthylthiomethyl-, 3-n-Propylthiomethyl-, 3-iso-Propylthiomethyl-, 3-Methylsulfinylmethyl-, 3-Äthylsulfinylmethyl-, 3-n-Propylsulfinylmethyl-, 3-iso-Propylsulfinylmethyl-, 3-Methylsulfonylmethyl-, 3-Äthylsulfonylmethyl-, 3-n-Propylsulfonylmethyl- und 3-iso-Propylsulfonylmethyl-4-methyl-, -4-äthyl-, 4-n-propyl- und -4-iso-propyl-1-cyclobutyl-5-hydroxy-pyrazol ;

3-Methoxy-methyl-, 3-Äthoxymethyl-, 3-n-Propoxymethyl-, 3-iso-Propoxymethyl-, 3-Methylthiomethyl-, 3-Äthylthiomethyl-, 3-n-Propylthiomethyl-, 3-iso-Propylthiomethyl-, 3-Methysulfinylmethyl-, 3-Äthylsulfinylmethyl-, 3-n-Propylsulfinylmethyl-, 3-iso-Propylsulfinylmethyl-, 3-Methylsulfonylmethyl-, 3-Äthylsulfonylmethyl-, 3-n-Propylsulfonylmethyl- und 3-iso-Propylsulfonylmethyl-4-methyl-, -4-äthyl-, -4-n-propyl- und -4-iso-propyl-1-cyclobutyl-5-hydroxy-pyrazol, sowie

3-Methoxymethyl-, 3-Äthoxymethyl-, 3-n-Propoxymethyl-, 3-iso-Propoxymethyl-, 3-Methylthiomethyl-, 3-Äthylthiomethyl-, 3-n-Propylthiomethyl-, 3-iso-Propylthiomethyl-, 3-Methylsulfinylmethyl-, 3-Äthylsulfinylmethyl-, 3-n-Propylsulfinylmethyl-, 3-iso-Propylsulfinylmethyl-, 3-Methylsulfonylmethyl-, 3-Äthylsulfonylmethyl-, 3-n-Propylsulfonylmethyl- und 3-iso-Propylsulfonylmethyl-4-methyl-, 4-äthyl-, -4-n-propyl- und -4-iso-propyl-1-cyclohexyl-5-hydroxy-pyrazol.

Als Beispiel für die bei Verfahren (a) zu verwendenden Carbaminsäurehalogenide der Formel (III) sei N,N-Dimethyl-carbaminsäurechlorid genannt. Es ist, ebenso wie die bei Verfahren (b) einzusetzenden Reaktionskomponenten Phosgen und Dimethylamin, lange bekannt.

Die bei den Verfahren (c) und (d) als Ausgangsstoffe zu verwendenden N,N-Dimethyl-O-(3-alkylthiomethyl-pyrazol-(5)yl)-carbaminsäureester sind durch die Formel (I) mit der Maßgabe, daß $R^1$ für Alkylthio steht, definiert.

Vorzugsweise stehen darin R, $R^1$ und $R^2$ für diejenigen Reste, welche bei der Definition der Reste R, $R^1$ und $R^2$ in Formel (I) als bevorzugt genannt wurden, wobei jedoch $R^1$ nur für Alkylthio steht.

Als Beispiele für diese Ausgangsverbindungen seien genannt :

O-(1-Methyl-3-methylthiomethyl-4-methyl-pyrazol(5)yl)-,
O-(1-Methyl-3-äthylthiomethyl-4-methyl-pyrazol(5)yl)-,
O-(1-Methyl-3-n-propylthiomethyl-4-methyl-pyrazol(5)yl)-,
O-(1-Methyl-3-iso-propylthiomethyl-4-methyl-pyrazol(5)yl)-,
O-(1-Athyl-3-methylthiomethyl-4-methyl-pyrazol(5)yl)-,
O-(1-Äthyl-3-äthylthiomethyl-4-methyl-pyrazol(5)yl)-,
O-(1-Äthyl-3-n-propylthiomethyl-4-methyl-pyrazol(5)yl)-,
O-(1-Äthyl-3-iso-propylthiomethyl-4-methyl-pyrazol(5)yl)-,
O-(1-n-Propyl-3-methylthiomethyl-4-methyl-pyrazol(5)yl)-,
O-(1-n-Propyl-3-ethylthiomethyl-4-methyl-pyrazol(5)yl)-,
O-(1-n-Propyl-3-n-propylthiomethyl-4-methyl-pyrazol(5)yl)-,
O-(1-n-Propyl-3-iso-propylthiomethyl-4-methyl-pyrazol(5)-yl)-,
O-(1-iso-Propyl-3-methylthiomethyl-4-methyl-pyrazol(5)yl)-,
O-(1-iso-Propyl-3-äthylthiomethyl-4-methyl-pyrazol(5)yl)-,
O-(1-iso-Propyl-3-n-propylthiomethyl-4-methyl-pyrazol(5)-yl)-,
O-(1-iso-Propyl-3-iso-propylthiomethyl-4-methyl-pyrazol-(5)yl)-,
O-(1-(2-Cyanoäthyl)-3-methylthiomethyl-4-methyl-pyrazol-(5)yl)-,
O-(1-(2-Cyano-äthyl)-3-äthylthiomethyl-4-methyl-pyrazol-(5)yl)-,
O-(1-(2-Cyano-äthyl)-3-n-propylthiomethyl-4-methyl-pyrazol(5)yl)-,
O-(1-(2-Cyano-äthyl)-3-iso-propylthiomethyl-4-methyl-pyrazol(5)yl)-,
O-(1-Cyclopropyl-3-methylthiomethyl-4-methyl-pyrazol(5)yl)-,
O-(1-Cyclopropyl-3-äthylthiomethyl-4-methyl-pyrazol(5)yl)-,
O-(1-Cyclopropyl-3-n-propylthiomethyl-4-methyl-pyrazol-(5)yl)-,
O-(1-Cyclopropyl-3-iso-propylthiomethyl-4-methyl-pyrazol-(5)yl)-,
O-(1-Cyclopentyl-3-methylthiomethyl-4-methyl-pyrazol(5)yl)-,
O-(1-Cyclopentyl-3-äthylthiomethyl-4-methyl-pyrazol(5)yl)-,
O-(1-Cyclopentyl-3-n-propylthiomethyl-4-methyl-pyrazol-(5)yl)-,
O-(1-Cyclopentyl-3-iso-propylthiomethyl-4-methyl-pyrazol-(5)yl)-,
O-(1-Cyclohexyl-3-methylthiomethyl-4-methyl-pyrazol(5)yl)-,
O-(1-Cyclohexyl-3-äthylthiomethyl-4-methyl-pyrazol(5)yl)-,
O-(1-Cyclohexyl-3-n-propylthiomethyl-4-methyl-pyrazol(5)yl)-,

O-(1-Cyclohexyl-3-iso-propylthiomethyl-4-methyl-pyrazol-(5)yl)-,
O-(1-iso-Propyl-3-methylthiomethyl-4-äthyl-pyrazol(5)yl)-,
O-(1-iso-Propyl-3-methylthiomethyl-4-n-propyl-pyrazol(5)yl)-,
O-(1-iso-Propyl-3-methylthiomethyl-4-iso-propyl-pyrazol-(5)yl)-,
O-(1-(2-Cyano-äthyl)-3-methylthiomethyl-4-äthyl-pyrazol-(5)yl)- und
O-(1-(2-Cyano-äthyl)-3-methylthiomethyl-4-n-propyl-pyrazol-(5)yl)- -N,N-dimethyl-carbaminsäureester.

Diese als Ausgangsstoffe einzusetzenden Alkylthioverbindungen der Formel (I) können aus Verbindungen der Formel (II), worin $R^1$ für Alkylthio steht, nach Herstellungsvariante (a) oder (b) hergestellt werden.

Die in Verfahren (c) bzw. (d) zu verwendenden Oxidationsmittel Wasserstoffperoxid bzw. m-Chlorperbenzoesäure sind bekannte Verbindungen.

Die Verfahren (a), (b), (c) und (d) zur Herstellung der neuen N,N-Dimethyl-O-pyrazolyl-carbaminsäureester werden bevorzugt unter Verwendung von Verdünnungsmitteln durchgeführt. Als solche kommen praktisch alle inerten organischen Lösungsmittel infrage. Hierzu gehören insbesondere aliphatische und aromatische, gegebenenfalls chlorierte Kohlenwasserstoffe, wie Benzin, Benzol, Toluol, Xylol, Methylenchlorid, Chloroform, Tetrachlorkohlenstoff, Chlorbenzol und o-Dichlorbenzol, Äther wie Diäthyl- und Dibutyläther, Tetrahydrofuran und Dioxan, Ketone wie Aceton, Methyläthyl-, Methylisopropyl- und Methylisobutylketon sowie Nitrile wie Acetonitril und Propionitril.

Für Verfahren (c) werden aliphatische Carbonsäuren, wie z.B. Essigsäure, als Lösungsmittel bevorzugt. Verfahren (a) und (b) werden bevorzugt unter Verwendung von Säureakzeptoren durchgeführt. Als Säureakzeptoren können alle üblichen Säurebindemittel Verwendung finden. Besonders bewährt haben sich Alkalicarbonate und -alkoholate wie Natrium- und Kaliumcarbonat, Natrium- und Kaliummethylat bzw. -äthylat, ferner aliphatische, aromatische oder heterocyclische Amine, beispielsweise Triäthylamin, Trimethylamin, Dimethylanilin, Dimethylbenzylamin und Pyridin.

Die erfindungsgemäßen Verfahren werden im allgemeinen bei Temperaturen zwischen 0 und 100 °C durchgeführt. Bevorzugt wird für Verfahren (a) und (b) der Bereich zwischen 20 und 80 °C, für Verfahren (c) und (d) der Bereich zwischen 0 und 25 °C. Die Umsetzungen werden im allgemeinen bei Normaldruck durchgeführt. Zur Durchführung von Verfahren (a) und (b) werden die Ausgangsstoffe gewöhnlich in äquimolaren Mengen eingesetzt. Ein Überschuß der einen oder anderen Reaktionskomponente bringt keine wesentlichen Vorteile. Die Umsetzung wird im allgemeinen in einem geeigneten Verdünnungsmittel in Gegenwart eines Säureakzeptors durchgeführt. Nach Reaktionsende gießt man die Mischung in Wasser und schüttelt mit einem organischen Lösungsmittel, z.B. Toluol aus. Die organische Phase wird dann wie üblich durch Waschen, Trocknen und Abdestillieren des Lösungsmittels aufgearbeitet.

Bei Verfahren (c) werden die Reaktionspartner ebenfalls bevorzugt in äquimolaren Mengen eingesetzt. Die in der Regel hierbei als Verdünnungsmittel verwendete Essigsäure wird nach Reaktionsende in Vakuum abdestilliert.

Danach gibt man ein organisches Lösungsmittel, z.B. Methylenchlorid zu und arbeitet die organische Phase wie üblich durch Waschen, Trocknen und Abdestillieren des Lösungsmittels auf.

Bei Verfahren (d) wird die als Oxidationsmittel verwendete m-Chlor-perbenzoesäure gewöhnlich im Überschuß eingesetzt, und zwar zwischen 2 und 3 Mol je Mol O-(3-Alkylthiomethyl-pyrazol(5)yl)-N,N-dimethylcarbaminsäureester. Die Umsetzung wird gewöhnlich in einem mit Wasser nicht mischbaren Lösungsmittel durchgeführt. Man wäscht dann neutral und arbeitet wie unter (a) und (c) beschrieben auf.

Die neuen Verbindungen fallen zum Teil in Form von Ölen an, die sich zum Teil nicht unzersetzt destillieren lassen, jedoch durch sogenanntes « Andestillieren », d.h. durch längeres Erhitzen unter vermindertem Druck auf mäßig erhöhte Temperaturen von den letzten flüchtigen Anteilen befreit und auf diese weise gereinigt werden. Zu ihrer Charakterisierung dient der Brechungsindex.

Soweit die neuen Produkte nach dem Abdestillieren des Lösungsmittels in fester Form anfallen, werden sie durch Umkristallisation gereinigt. Zur Charakterisierung dient der Schmelzpunkt.

Die Wirkstoffe eignen sich bei guter Pflanzenverträglichkeit und günstiger Warmblütertoxizität zur Bekämpfung von tierischen Schädlingen, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen genören :

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp.

Aus der Ordnung der Anoplura z.B. Phylloxera vastatrix, Pemphigus spp., Pediculus humanus corporis, Haematopinus spp., Lignognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp. Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp.

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage : Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser ; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid ; als feste Trägerstoffe kommen in Frage : z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate ; als feste Trägerstoffe für Granulate kommen in Frage : z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel ; als Emulgier- und/oder schaumerzeugende Mittel kommen in Frage : z.B. nichtionogenen und anionische Emulgatoren, wie Polyoxyäthylen-Fettsäure-Ester, Polyoxyäthylen-Fettalkohol-

Äther, z.B. Alkylarylpolyglykol-äther, Alkylsulfonate, Alkylsulfate, Arylsulfonate sowie Eiweißhydrolysate ; als Dispergiermittel kommen in Frage : z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die Anwendung der erfindungsgemäßen Wirkstoffe erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,000 000 1 bis zu 100 Gew.-% Wirkstoff, vorzugsweise zwischen 0,01 und 10 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

## Beispiel A

**Myzus-Test**

Lösungsmittel : 3 Gewichtsteile Dimethylformamid

Emulgator : 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea), die stark von der Pfirsichblattlaus (Myzus persicae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Blattläuse abgetötet wurden ; 0 % bedeutet, daß keine Blattläuse abgetötet wurden.

Bei diesem Test zeigen z.B. die folgenden Verbindungen der Herstellungsbeispiele überlegene Wirksamkeit gegenüber dem Stand der Technik : 1, 2, 3, 4, 5, 8, 9, 11, 12.

## Beispiel B

Grenzkonzentrations-Test/Wurzelsystemische Wirkung

Testinsekt : Myzus persicae

Lösungsmittel : 3 Gewichtsteile Aceton

Emulgator : 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik : 4, 5, 11, 12, 1, 8, 9, 2, 3.

## Herstellungsbeispiele

### Beispiel 1

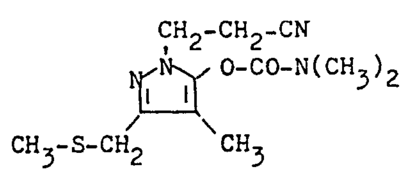

Ein Gemisch aus 10,5 g (50 nMol) 1-(2-Cyanäthyl)-3-methyl-thiomethyl-4-methyl-5-hydroxy-pyrazol, 8,4 g (60 nMol) Kaliumcarbonat, 200 ml Acetonitril und 5,4 g (50 nMol) N,N-Dimethyl-carbaminsäurechlorid wird 12 Stunden bei 50 °C gerührt. Nach Zugabe von 200 ml Wasser extrahiert man mit 300 ml Toluol. Die organische Phase wird über Natriumsulfat getrocknet und im Vakuum eingedampft. Zurück bleiben 12 g (85 % der Theorie) N,N-Dimethyl-O-(1-(2-cyanäthyl)-3-methylthiomethyl-4-methylpyrazol(5)yl)-carbaminsäureester in Form farbloser Kristalle mit dem Schmelzpunkt 78 °C.

Beispiel 2

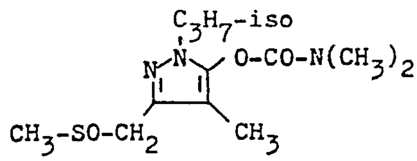

Eine Lösung von 13,6 g (0,05 Mol) N,N-Dimethyl-O-(1-isopropyl-3-methylthiomethyl-4-methyl-pyrazol(5)yl)-carbaminsäureester in 50 ml Eisessig wird bei 5-10 °C mit 3,4 g (0,05 Mol) 50 %igem Wasserstoffperoxid versetzt. Man rührt das Gemisch 6 Stunden bei Raumtemperatur nach und destilliert dann das Lösungsmittel in Vakuum ab. Der Rückstand wird in 100 ml Methylenchlorid gelöst und mit einer Lösung von 10 g Kaliumcarbonat in 15 ml Wasser gewaschen. Die organische Phase wird abgetrennt und über Natriumsulfat getrocknet. Dann destilliert man das Lösungsmittel in Vakuum ab. Man erhält so 12,2 g (85 % der Theorie) N,N-Dimethyl-O-(1-iso-propyl-3-methyl-sulfinylmethyl-4-methyl-pyrazol(5)yl)-carbaminsäureester in Form eines braunen Öles mit dem Brechungsindex $n_D^{20}$ : 1,522 2.

Beispiel 3

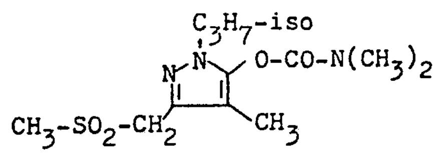

Zu einer Lösung von 13,6 g (0,05 Mol) N,N-Dimethyl-O-(1-iso-propyl-3-methylthiomethyl-4-methyl-pyrazol(5)yl)-carbaminsäureester in 50 ml Chloroform tropft man bei 5 °C eine Lösung von 21,3 g m-Chlorperbenzoesäure in 150 ml Chloroform. Das Gemisch wird über Nacht bei Raumtemperatur nachgerührt und dann filtriert. Man wäscht das Filtrat mit 10 ml konzentrierter Kaliumcarbonatlösung und trocknet es über Natriumsulfat. Dann wird das Lösungsmittel in Vakuum abgezogen. Zurück bleiben 12,2 g (82 % der Theorie) N-N-Dimethyl-O-(1-iso-propyl-3-methylsulfonylmethyl-4-methyl-pyrazol-(5)yl)-carbaminsäureester in Form beiger Kristalle mit dem Schmelzpunkt 92 °C.

Analog einem der Beispiele 1 bis 3 können die folgenden Verbindungen der Formel Ia

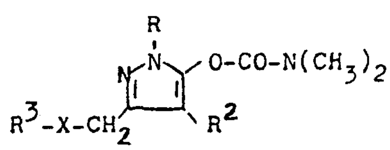

(Ia)

hergestellt werden :

| Beispiel Nr. | R | $R^3$ | $R^2$ | X | Ausbeute (% der Theorie) | Schmelzpunkt [°C] Brechungsindex; |
|---|---|---|---|---|---|---|
| 4 | $CH_3$ | $CH_3$ | $CH_3$ | S | 75 | 77 |
| 5 | $C_3H_7$-iso | $CH_3$ | $CH_3$ | S | 93 | $n_D^{20}$:1,5149 |

(Fortsetzung)

| Beispiel Nr. | R | $R^3$ | $R^2$ | X | Ausbeute (% der Theorie) | Schmelzpunkt(°C); Brechungsindex; |
|---|---|---|---|---|---|---|
| 6 | $CH_2-CH_2-CN$ | $CH_3$ | $CH_3$ | SO | 98 | 129 |
| 7 | $CH_2-CH_2-CN$ | $CH_3$ | $CH_3$ | $SO_2$ | 80 | 168 |
| 8 | $C_3H_7$-iso | $CH_3$ | $CH_3$ | O | 79 | $n_D^{20}$:1,4850 |
| 9 | $C_3H_7$-iso | $C_2H_5$ | $CH_3$ | S | 92 | $n_D^{20}$:1,5090 |
| 10 | $C_3H_7$-iso | $C_3H_7$-n | $CH_3$ | S | | |
| 11 | $C_3H_7$-iso | $CH_3$ | $C_2H_5$ | S | 80 | $n_D^{20}$:1,5112 |
| 12 | $C_3H_7$-iso | $CH_3$ | $C_3H_7$-n | S | 91 | $n_D^{20}$:1,5074 |
| 13 | $C_2H_5$ | $CH_3$ | $CH_3$ | S | | |
| 14 | $C_3H_7$-n | $CH_3$ | $CH_3$ | S | | |
| 15 | 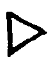 | $CH_3$ | $CH_3$ | S | | |
| 16 | 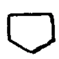 | $CH_3$ | $CH_3$ | S | | |
| 17 | 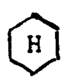 | $CH_3$ | $CH_3$ | S | | |
| 18 | $C_4H_9$-sek. | $CH_3$ | $CH_3$ | S | | |
| 19 | $C_3H_7$-iso | $CH_3$ | $C_3H_7$-iso | S | | |
| 20 | $C_3H_7$-iso | $C_3H_7$-iso | $CH_3$ | S | | |
| 21 | 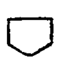 | $CH_3$ | $CH_3$ | SO | | |
| 22 | 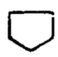 | $CH_3$ | $CH_3$ | $SO_2$ | | |
| 23 | $CH_2-CH_2-CN$ | $C_2H_5$ | $CH_3$ | S | | |
| 24 | $CH_2-CH_2-CN$ | $CH_3$ | $C_2H_5$ | S | | |
| 25 | $CH_2-CH_2-CN$ | $CH_3$ | $C_3H_7$-n | S | | |
| 26 | 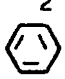 | $CH_3$ | $CH_3$ | O | | |
| 27 | 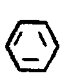 | $CH_3$ | $CH_3$ | S | 82 | 68 |

Die als Ausgangsmaterialien einzusetzenden Verbindungen können z.B. wie folgt hergestellt werden :

Beispiel a

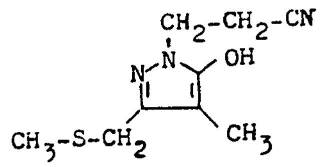

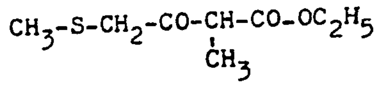 :

1. Stufe :

Eine Lösung von 17,6 g (0,1 Mol) 4-Methylthioacetessigsäureäthylester in 100 ml Tetrahydrofuran wird unter Kühlen zunächst mit 12,3 g (0,11 Mol) Kalium-tert.-butanolat und dann mit 15,6 g (0,11 Mol) Methyljodid versetzt. Man rührt das Gemisch 12 Stunden bei 60 °C nach, destilliert dann das Lösungsmittel im Vakuum ab, versetzt den Rückstand mit 200 ml Methylenchlorid und schüttelt die Lösung zwei mal mit je 100 ml Wasser aus. Die organische Phase wird über Natriumsulfat getrocknet und im Vakuum eingedampft. Der Rückstand wird im Vakuum destilliert. Man erhält so 14 g (74 % der Theorie) 2-Methyl-4-methylthio-acetessigsäureäthylester mit dem Siedepunkt 95-99 °C/2 mm.

2. Stufe :

Eine Mischung aus 38 g (0,2 Mol) 2-Methyl-4-methylthioacetessigsäureäthylester, 17 g (0,2 Mol) 2-Cyanäthylhydrazin und 100 ml Methanol wird 6 Stunden bei 60 °C gerührt. Dann destilliert man das Lösungsmittel im Vakuum ab und verreibt den Rückstand mit Petroläther.

Nach Kristallisation wird abgesaugt. Man erhält auf diese Weise 37 g (88 % der Theorie) 1-(2-Cyanäthyl)-3-methylthiomethyl-4-methyl-5-hydroxypyrazol als beiges Pulver mit dem Schmelzpunkt 98 °C.

Analog Beispiel a können die folgenden Verbindungen der Formel

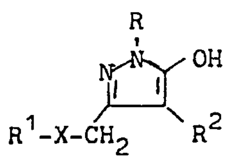

hergestellt werden :

| Beispiel | R | $R^3$ | $R^2$ | X | Ausbeute (% der Theorie) | Schmelzpunkt (°C) |
|---|---|---|---|---|---|---|
| b | $CH_3$ | $CH_3$ | $CH_3$ | S | 70 | 67 |
| c | $C_3H_7$-iso | $CH_3$ | $CH_3$ | S | 47 | 84 |
| d | $C_3H_7$-iso | $CH_3$ | $CH_3$ | O | 76 | 78 |
| e | $C_3H_7$-iso | $C_2H_5$ | $CH_3$ | S | 57 | 117 |
| f | $C_3H_7$-iso | $C_3H_7$-n | $CH_3$ | S | | |
| g | $C_3H_7$-iso | $CH_3$ | $C_2H_5$ | S | 72 | zähflüssig |
| h | $C_3H_7$-iso | $CH_3$ | $C_3H_7$-n | S | 57 | 79 |

(Fortsetzung)

| Beispiel | R | $R^3$ | $R^2$ | X | Ausbeute (% der Theorie) | Schmelzpunkt (°C) |
|---|---|---|---|---|---|---|
| i | $C_2H_5$ | $CH_3$ | $CH_3$ | S | | |
| j | $C_3H_7$-n | $CH_3$ | $CH_3$ | S | | |
| k | 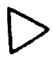 | $CH_3$ | $CH_3$ | S | | |
| l | 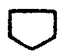 | $CH_3$ | $CH_3$ | S | | |
| m | 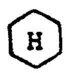 | $CH_3$ | $CH_3$ | S | | |
| n | $C_4H_9$-sek. | $CH_3$ | $CH_3$ | S | | |
| o | $C_3H_7$-iso | $CH_3$ | $C_3H_7$-iso | S | | |
| p | $C_3H_7$-iso | $C_3H_7$-iso | $CH_3$ | S | | |
| q | $CH_2$-$CH_2$-CN | $C_2H_5$ | $CH_3$ | S | | |
| r | $CH_2$-$CH_2$-CN | $CH_3$ | $C_2H_5$ | S | | |
| s | 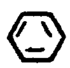 | $CH_3$ | $CH_3$ | O | | |
| t | 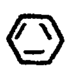 | $CH_3$ | $CH_3$ | S | 80 | 117 |

**Ansprüche**

1. N,N-Dimethyl-O-pyrazolyl-carbaminsäureester der Formel

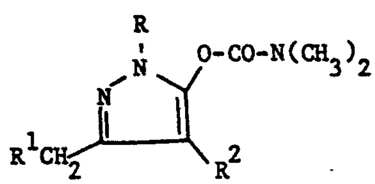

(I)

in welcher

R für Alkyl, Cyanoäthyl, Cycloalkyl oder Phenyl steht,

$R^1$ für Alkoxy, Alkylthio, Alkylsulfinyl oder Alkylsulfonyl steht und

$R^2$ für Alkyl steht.

2. Verfahren zur Herstellung der N,N-Dimethyl-O-pyrazolyl-carbaminsäureester der Formel (I)

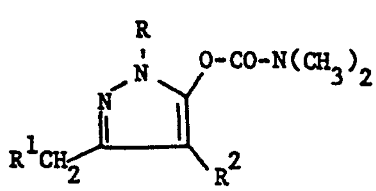

in welcher
R für Alkyl, Cyanoäthyl oder Phenyl steht,
$R^1$ für Alkoxy, Alkylthio, Alkylsulfinyl oder Alkylsulfonyl steht und
$R^2$ für Alkyl steht,
dadurch gekennzeichnet, daß man
a) Hydroxy-pyrazole der Formel II

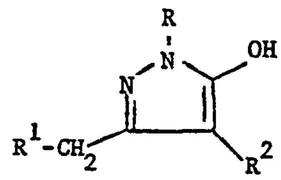

(II)

in welcher
R, $R^1$ und $R^2$ die oben angegebene Bedeutung haben, mit N,N-Dimethyl-carbaminsäurehalogeniden der Formel III

$$Hal—CO—N(CH_3)_2 \quad (III)$$

in welcher
Hal für Chlor oder Brom steht, gegebenenfalls in Gegenwart eines Säureakzeptors und gegebenenfalls unter Verwendung eines inerten Verdünnungsmittels umsetzt, oder

b) 5-Hydroxy-pyrazole der Formel (II), in welcher R, $R^1$ und $R^2$ die oben angegebene Bedeutung haben, mit Phosgen und anschließend mit Dimethylamin, gegebenenfalls in Gegenwart eines Säureakzeptors und gegebenenfalls unter Verwendung eines inerten Verdünnungsmittels umsetzt, oder für den Fall, daß Verbindungen der Formel (I), in welcher R und $R^2$ die oben angegebene Bedeutung haben und $R^1$ für Alkylsulfinyl steht, erhalten werden sollen, indem man

c) N,N-Dimethyl-O-(3-alkylthiomethyl-pyrazol(5)yl)-carbaminsäureester der Formel (I), in welcher R und $R^2$ die oben angegebene Bedeutung haben und $R^1$ für Alkylthio steht, mit mindestens der äquimolaren Menge Wasserstoffperoxid, gegebenenfalls unter Verwendung eines Verdünnungsmittels umsetzt oder für den Fall, daß Verbindungen der Formel (I), in welcher R und $R^2$ die oben angegebene Bedeutung haben und $R^1$ für Alkylsulfonyl steht, erhalten werden sollen, indem man

d) N,N-Dimethyl-O-(3-alkylthiomethyl-pyrazol(5)yl)-carbaminsäureester der Formel (I), in welcher R und $R^2$ die oben angegebene Bedeutung haben und $R^1$ für Alkylthio steht, mit wenigstens zwei Moläquivalenten m-Chlor-perbenzoesäure, gegebenenfalls in. Gegenwart eines Verdünnungsmittels umsetzt.

3. Schädlingsbekämpfungsmittel, gekennzeichnet durch einen Gehalt an mindestens einem N,N-Dimethyl-O-pyrazolyl-carbaminsäureester der Formel (I).

4. Insektizide und/oder akariziede und/oder nematizide Mittel, gekennzeichnet durch einen Gehalt an mindestens einem N,N-Dimethyl-O-pyrazolyl-carbaminsäureester der Formel (I).

5. Verwendung von N,N-Dimethyl-O-pyrazolyl-carbaminsäureester der Formel (I) zur Bekämpfung von Insekten und/oder Spinnentieren und/oder Nematoden.

6. Verfahren zur Bekämpfung von Insekten und/oder Spinnentieren und/oder Nematoden, dadurch gekennzeichnet, daß man N,N-Dimethyl-O-pyrazolyl-carbaminsäureester der Formel (I) auf Insekten und/oder Spinnentieren und/oder Nematoden und/oder ihren Lebensraum einwirken läßt.

7. Verfahren zur Herstellung insektizider und/oder akarizider und/oder nematizider Mittel, dadurch gekennzeichnet, daß man N,N-Dimethyl-O-pyrazolyl-carbaminsäureester der Formel (I) mit Streckmitteln und/oder oberflächenaktiven Mitteln vermischt.

**Claims**

1. N,N-Dimethyl-carbamic acid O-pyrazolyl esters of the formula

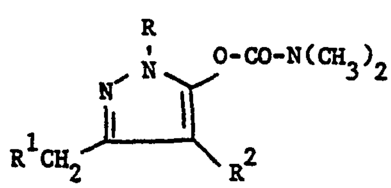

(I)

in which
R represents alkyl, cyanoethyl, cycloalkyl or phenyl,
$R^1$ represents alkoxy, alkylthio, alkylsulphinyl or alkylsulphonyl and

$R^2$ represents alkyl.

2. Process for the preparation of the N,N-dimethylcarbamic acid O-pyrazolyl esters of the formula (I)

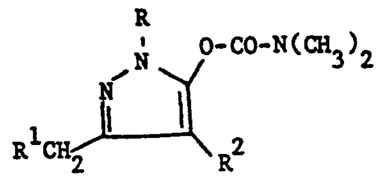

in which

R represents alkyl, cyanoethyl, cycloalkyl or phenyl,

$R^1$ represents alkoxy, alkylthio, alkylsulphinyl or alkylsulphonyl and

$R^2$ represents alkyl,

characterised in that

a) hydroxy-pyrazoles of the formula II

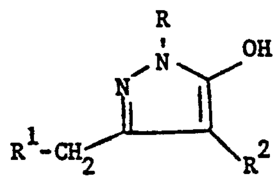

(II)

in which

R, $R^1$ and $R^2$ have the meaning indicated above, are reacted with N,N-dimethyl-carbamic acid halides of the formula III

$$Hal—CO—N(CH_3)_2 \qquad (III)$$

in which

Hal represents chlorine or bromine, if appropriate in the presence of an acid acceptor and if appropriate using an inert diluent, or

b) 5-hydroxy-pyrazoles of the formula (II) in which R,$R^1$ and $R^2$ have the meaning indicated above, are reacted with phosgene and the product is then reacted with dimethylamine, if appropriate in the presence of an acid acceptor and if appropriate using an inert diluent, or, in the case where compounds of the formula (I) in which R and $R^2$ have the meaning indicated above and $R^1$ represents alkylsulphinyl, are to be obtained, by a procedure in which

c) N,N-dimethyl-carbamic acid O-(3-alkylthiomethyl-pyrazol-5-yl) esters of the formula (I) in which R and $R^2$ have the meaning indicated above and $R^1$ represents alkylthio, are reacted with at least an equimolar amount of hydrogen peroxide, if appropriate using a diluent, or, in the case where compounds of the formula (I) in which R and $R^2$ have the meaning indicated above and $R^1$ represents alkylsulphonyl, are to be obtained, by a procedure in which

d) N,N-dimethyl-carbamic acid O-(3-alkylthiomethyl-pyrazol-5-yl) esters of the formula (I) in which R and $R^2$ have the meaning indicated above and $R^1$ represents alkylthio, are reacted with at least two molar equivalents of m-chloro-perbenzoic acid, if appropriate in the presence of a diluent.

3. Agents for combating pests, characterised in that they contain at least one N,N-dimethyl-carbamic acid O-pyrazolyl ester of the formula (I).

4. Insecticidal and/or acaricidal and/or nematicidal agents, characterised in that they contain at least one N,N-dimethyl-carbamic acid O-pyrazolyl ester of the formula (I).

5. Use of N,N-dimethyl-carbamic acid O-pyrazolyl esters of the formula (I) for combating insects and/or arachnidae and/or nematodes.

6. Process for combating insects and/or arachnidae and/or nematodes, characterised in that N,N-dimethyl-carbamic acid O-pyrazolyl esters of the formula (I) are allowed to act on insects and/or arachnidae and/or nematodes and/or their environment.

7. Process for the preparation of insecticidal and/or acaricidal and/or nematicidal agents, characterised in that N,N-dimethyl-carbamic acid O-pyrazolyl esters of the formula (I) are mixed with extenders and/or surface-active agents.

**Revendications**

1. Esters N,N-diméthyl-O-pyrazolyl-carbamiques de formule

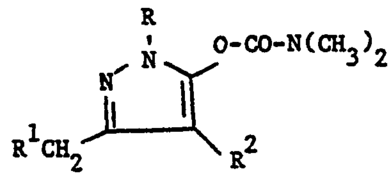

(I)

dans laquelle

R représente un groupe alkyle, cyanéthyle, cycloalkyle ou phényle,

$R^1$ représente un groupe alcoxy, alkylthio, alkylsulfinyle ou alkylsulfonyle et

$R^2$ représente un groupe alkyle.

2. Procédé de préparation des esters N,N-diméthyl-O-pyrazolyl-carbamiques de formule (I)

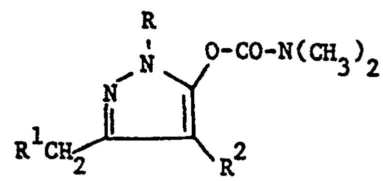

dans laquelle

R représente un groupe alkyle, cyanéthyle, cycloalkyle ou phényle,

$R^1$ représente un groupe alcoxy, alkylthio, alkylsulfinyle ou alkylsulfonyle et

$R^2$ représente un groupe alkyle,

caractérisé en ce que :

a) on fait réagir des hydroxy-pyrazoles de formule II

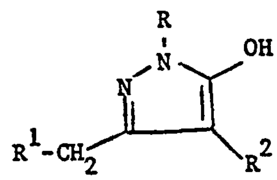

(II)

dans laquelle

R, $R^1$ et $R^2$ ont les significations indiquées ci-dessus, avec des halogénures d'acides N,N-diméthyl-carbamiques de formule III

$$Hal—CO—N(CH_3)_2 \qquad (III)$$

dans laquelle

Hal représente le chlore ou le brome, éventuellement en présence d'un accepteur d'acide et le cas échéant en utilisant un diluant inerte, ou bien

b) on fait réagir des 5-hydroxy-pyrazoles de formule II, dans laquelle R, $R^1$ et $R^2$ ont les significations indiquées ci-dessus, avec le phosgène puis avec la diméthylamine, éventuellement en présence d'un accepteur d'acide et le cas échéant en utilisant un diluant inerte, ou bien, dans le cas où l'on doit obtenir des composés de formule I, dans laquelle R et $R^2$ ont les significations indiquées ci-dessus et $R^1$ représente un groupe alkylsulfinyle,

c) on fait réagir des esters N,N-diméthyl-O-(3-alkylthiométhyl-pyrazol(5)yl)-carbamiques de formule I dans laquelle R et $R^2$ ont les significations indiquées ci-dessus et $R^1$ représente un groupe alkylthio avec une quantité au moins équimoléculaire de peroxyde d'hydrogène, éventuellement en utilisant un diluant, ou bien, dans le cas où l'on doit obtenir des composés de formule I dans laquelle R et $R^2$ ont les significations indiquées ci-dessus et $R^1$ représente un groupe alkylsulfonyle,

d) on fait réagir des esters N,N-diméthyl-O-(3-alkylthiométhyl-pyrazol(5)yl)-carbamiques de formule I dans laquelle R et $R^2$ ont les significations indiquées ci-dessus et $R^1$ représente un groupe alkylthio, avec au moins deux équivalents molaires d'acide m-chloro-perbenzoïque, éventuellement en présence d'un diluant.

3. Produit pesticide, caractérisé en ce qu'il contient au moins un ester N,N-diméthyl-O-pyrazolyl-carbamique de formule I.

4. Produit insecticide et/ou acaricide et/ou nématocide, caractérisé en ce qu'il contient au moins un ester N,N-diméthyl-O-pyrazolyl-carbamique de formule I.

5. Utilisation des esters N,N-diméthyl-O-pyrazolyl-carbamiques de formule I dans la lutte contre les insectes et/ou les acariens et/ou les nématodes.

6. Procédé pour combattre les insectes et/ou les acariens et/ou les nématodes, caractérisé en ce que

l'on fait agir des esters N,N-diméthyl-O-pyrazolyl-carbamiques de formule I sur les insectes et/ou les acariens et/ou les nématodes et/ou leur habitat.

7. Procédé de préparation de produits insecticides et/ou acaricides et/ou nématocides, caractérisé en ce que l'on mélange des esters N,N-diméthyl-O-pyrazolyl-carbamiques de formule I avec des diluants et/ou des agents tensio-actifs.